# EUROPEAN PATENT SPECIFICATION

(11) **EP 0 671 623 B1**
(45) Date of publication and mention of the grant of the patent: **07.07.1999**
(21) Application number: 95301612.8
(22) Date of filing: 09.03.1995
(51) Int. Cl.: G01N 27/04

(54) **Apparatus for measuring moisture content of grain or the like**
Messgerät zur Bestimmung der Feuchtigkeit von Körnern oder ähnlichem
Appareil de mesure de l'humidité de grains et semblables

(30) Priority: 09.03.1994 GB 9404596
(43) Date of publication of application: 13.09.1995
(73) Proprietor: PROTIMETER PLC, Marlow, Buckinghamshire SL7 1LX (GB)
(72) Inventor: Dadachanji, Fali Minocher, Buckinghamshire SL7 2DE (GB)
(74) Representative: Howden, Christopher Andrew

(56) References cited:
- DE-A- 2 941 063
- DE-A- 3 904 653
- DE-B- 1 129 323
- DE-C- 708 389
- GB-A- 885 306
- US-A- 3 293 907

## Description

THIS INVENTION relates to improved apparatus for the preparation of a material before moisture-content measurement using an electrical moisture meter.

One method of measuring moisture content in materials such as grain and other particulates electrically, is to measure the conductance or capacitance of the material. An established procedure for preparing a sample of the material before the measurement can be made is to grind (pulverize) the material into a uniform particle size and then as a separate step, to place or compress the ground material in good contact with a set of electrodes connected with an instrument for measuring capacitance or conductance.

The above procedure can be cumbersome and can lead to errors as there is the possibility of a change of moisture content during grinding and during subsequent transfer of the ground material to the measuring electrodes. Other procedures where the grain is mashed and compressed between two plates lead to difficulties at different moisture contents and different sample sizes.

British Patent Specification No. 885306 discloses apparatus for measuring the moisture content of grain and which comprises a body, a grinding mechanism in the body comprising an outer grinding ring fixed in the body and a central grinding wheel rotatable within the grinding ring. The body provides a chamber below the grinding mechanism to receive, directly from the grinding mechanism, grain ground by the grinding mechanism. A piston is carried on the underside of the central grinding wheel. The central grinding wheel is rotatable by means of a flat-sided spindle received within a slot extending diametrally across a driving shaft extending axially from a knob rotatable at an upper end of the body. The central grinding wheel is rotatable with respect to but is fixed axially relative to a hollow screw, screw-threadedly engaged in an insert carried in the housing. Locating pins carried at the ends of the divided driving spindle can engage in keyways provided in the interior of the screw when the knob is pulled axially into an extended position so that the rotation of the knob then causes the central grinding wheel and with it the piston to be advanced into the chamber to compress the ground material against electrodes used in sensing the electrical resistance of the ground compressed material, to determine moisture content.

U.S. Patent Specification No. 3293907 discloses testing apparatus for cyclically determining the moisture content of grain and in which apparatus for grinding grain, compressing the ground grain and testing the electrical-resistance of the ground grain is rotated continuously within a casing having an inlet opening for grain at an upper part thereof and a discharge opening for tested samples adjacent a bottom thereof. During rotation of the mechanism, an inlet of the rotating apparatus comes into register with the inlet opening in the casing to receive a charge of grain to be ground then, as the apparatus rotates, the charge of grain is ground, returned to an inlet neck section of the rotating mechanism after the radially outer end of that section has been closed by a first transversely sliding gate, the charge of the ground grain then being held captive in that inlet neck section by dosing a second transversely sliding gate disposed radially inwardly of the first. The charge of ground grain, thus held captive in the neck section between the transversely sliding gates, is subsequently compressed, during continued rotation of the apparatus, by radial movement of the radially outer part of the neck section, carrying the first gate, towards the radially inner part of the neck section carried by the radially inner part of the neck section. This radial movement is occasioned by engagement of the radially outer part with a cam within the housing. The electrical resistance of the sample is then tested, the gates withdrawn and the tested sample expelled through the discharge opening.

It is an object of the present invention to provide an improved apparatus for use in measuring moisture content by the above-noted method.

According to the invention there is provided apparatus for use in measuring the moisture content of grain or other particulate material, comprising a body (A), a grinding mechanism (C) in the body for grinding grain or other particulate material, a chamber (M) provided by the body and disposed to receive, directly from the grinding mechanism, ground grain or other material ground by the grinding mechanism, a piston or ram (D) movable into said chamber (M) to compress the ground material therein, and electrical contacts so disposed in said chamber or on said ram as to be in contact with the mass of compressed ground material, resulting from such movement into the chamber, at predetermined locations on said mass, whereby the moisture content of the material can be determined on the basis of the electrical resistance, between said electrodes, provided by said mass of compressed ground material, said grinding mechanism and said ram or piston being operable selectively by a common input member (H) characterised in that said grinding mechanism (C) comprises a fixed grinding disc, stationary in the body, and a rotatable grinding disc, said common input member comprising a hub and a manually operable handle (H) secured to the hub, said hub providing the driving connection between the handle and the rotatable grinding disc, said piston or ram (D) being carried by a shaft (T) extending along the rotary axis of the handle within a hollow barrel mounted coaxially within the hub, the barrel being coupled with a cap arranged coaxially within said hub, said hollow barrel forming an output member of said clutch, and wherein (a) the shaft (T) is screw-threadedly engaged in said hollow barrel and is axially slidable in, but it non-rotatable with respect to, a central guide member fixed with respect to said body, or (b) the shaft is guided for axial movement in said barrel but is non-rotatable therein and the shaft is screw-threadedly engaged in said central guide member;
whereby rotation of said barrel within said body causes axial movement of said shaft (T) and thus of said piston (D) relative to said chamber (M), and wherein said barrel can be selectively rotationally coupled with said handle and hub or decoupled therefrom, by means of a slide (G) slidable along said handle (H) inwardly towards the rotary axis of the handle, or outwardly away from said rotary axis, respectively to extend into a diametral slot provided across the top of said cap and to be removed from said slot, and thus respectively to couple the handle with said cap and decouple the handle from said cap.

An embodiment of the invention is described below by way of example with reference to the accompanying drawings, wherein:-
FIGURE 1 is a view in vertical section of an apparatus embodying the invention in one position thereof,
FIGURE 2 is a view similar to Figure 1 but showing the apparatus in another position thereof, and
FIGURE 3 is a perspective view of the apparatus of Figures 1 and 2, in conjunction with apparatus for sensing electrical resistance and displaying moisture content values calculated therefrom.

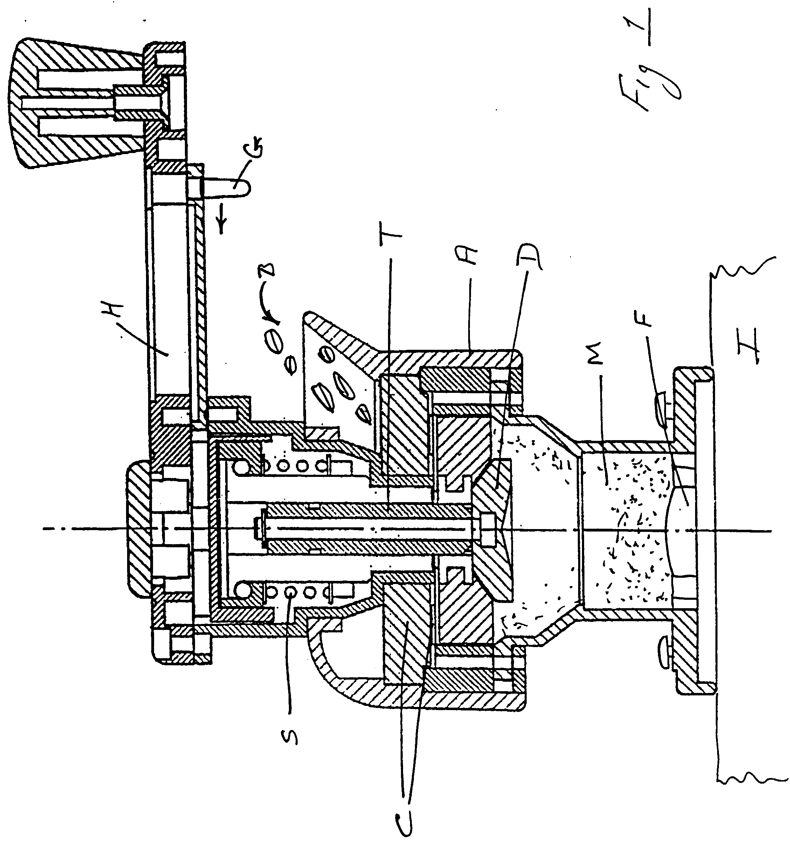

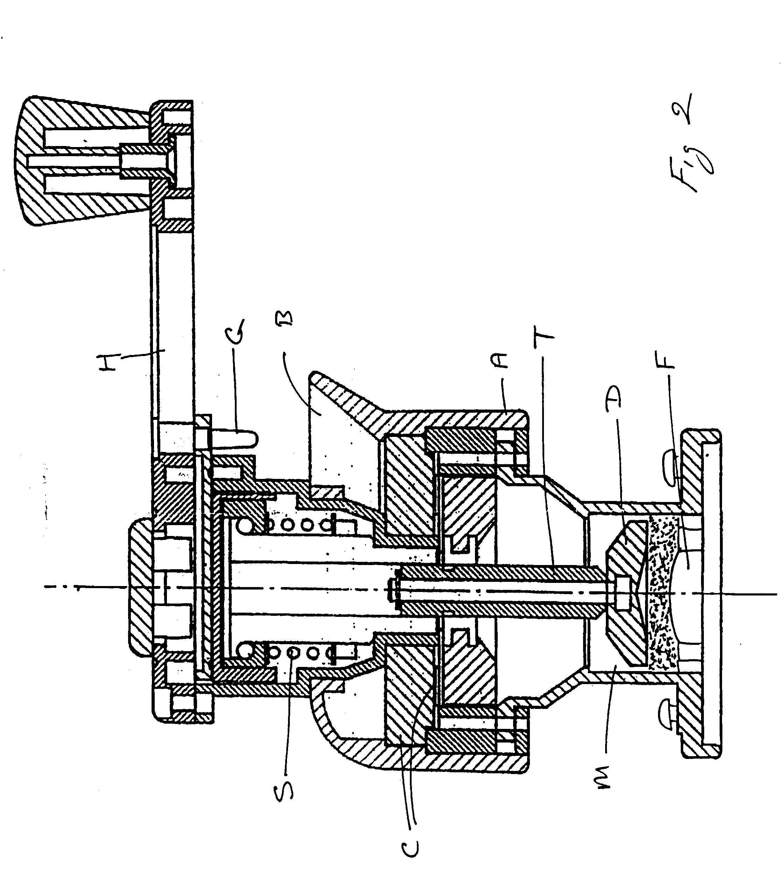

The apparatus shown in the drawings is intended for use in measurement of the moisture content of grain. The apparatus consists of the main body (A) affording a grain hopper (B). In use of the apparatus, grain introduced *via* the hopper is ground between the discs (C) formed with sharp blades or teeth, one of the discs being stationary in the body (A) and the other being coupled to a manually rotatable handle for rotation therewith. The handle (H) carries a slide (G) operable to couple the handle with, and decouple the handle from, a piston mechanism described below. With the slide (G) in the position shown in Figure 1, the piston mechanism (T, D) is de-coupled from the handle and is unaffected by turning action of the handle. With the slide (G) in the position shown in Figure 1, when the handle (H) is turned, the movable disc (C) rotates with respect to the fixed disc (C) and grain from the hopper is ground between the discs (C) and the ground grain falls into a measuring chamber (M) provided below the discs (C). When the chamber (M) is sufficiently full of grain, the slide (G) is placed as shown in Figure 2, it extends into a diametral slot provided across the top of a cap arranged co-axially within a hub secured to handle (H) and which provides the driving connection between the handle (H) and the rotatable grinding disc (C). The cap forms an input member of a slipping clutch which includes an output member in the form of a hollow internally screw-threaded barrel mounted within the hub coaxially with respect to the latter, in such a way that the hub can rotate around the hollow barrel when permitted to do so by the slipping clutch. The slipping clutch incorporates a friction ring or serrated ring which is non-rotatable with respect to the cap and is urged by the compression spring (S) surrounding the barrel into engagement with a friction ring or serrated ring carried by, or provided by the upper end of the barrel, so that the cap will slip relative to the barrel when the torque exerted by the cap on the barrel exceeds a predetermined value.

Located within the hollow barrel is an externally screw-threaded shaft (T) extending along the rotary axis of the handle and screw-threadedly engaged with the interior of the hollow barrel. The shaft (T) is slidable longitudinally with respect to discs (C) and housing (A) whilst being restrained from rotation with respect thereto, for example by longitudinal grooves (not shown) along the shaft (T) cooperating with splines (not shown) in a central guide member fixed within the fixed disc (C). Alternatively, of course, the hollow barrel and shaft (T) may have cooperating splines and grooves preventing relative rotation between the barrel and the shaft (T) and the shaft (T) may be screw-threadedly engaged with an internal screw thread in such central guide member. In either case the result is then when the shaft (T) is coupled directly to the handle (H) *via* the slipping clutch, when the handle (H) is turned, the shaft (T) is screwed downwards until such time as the slipping clutch mechanism allows the handle to slip without any further downward motion of shaft (T). A piston (D) is carried at the lower end of shaft (T) within chamber (M) and is dimensioned to be a close sliding fit in a cylindrical lower part of chamber (M). The bottom of chamber (M) is provided by a plate (F) of insulating material carrying spaced electrodes which are exposed to the chamber (M) and are connected with terminals (not shown) for connection with an electrical measuring instrument (I).

In operation, with the apparatus in the position shown in Figure 1, a measured amount of grain is poured into the hopper (B). The handle (H) is now turned clockwise, effectively grinding the grain between the plates (C). The ground grain falls into the measuring chamber (M). When all the grain has been ground through, the slide (G) is now pushed towards the hub, in the direction of the arrow in Figure 1, into the position shown in Figure 2, in which the slide, *via* the slipping clutch, directly couples the handle and the piston mechanism. Turning the handle (H) clockwise screws the shaft (T) and piston (D) downwards to compress the ground grain in chamber (M). The amount of compression of the grain is regulated by the force exerted by piston (D) which in turn is regulated by the action of the slipping clutch. The sample is now prepared ready for measurement with instrument (I).

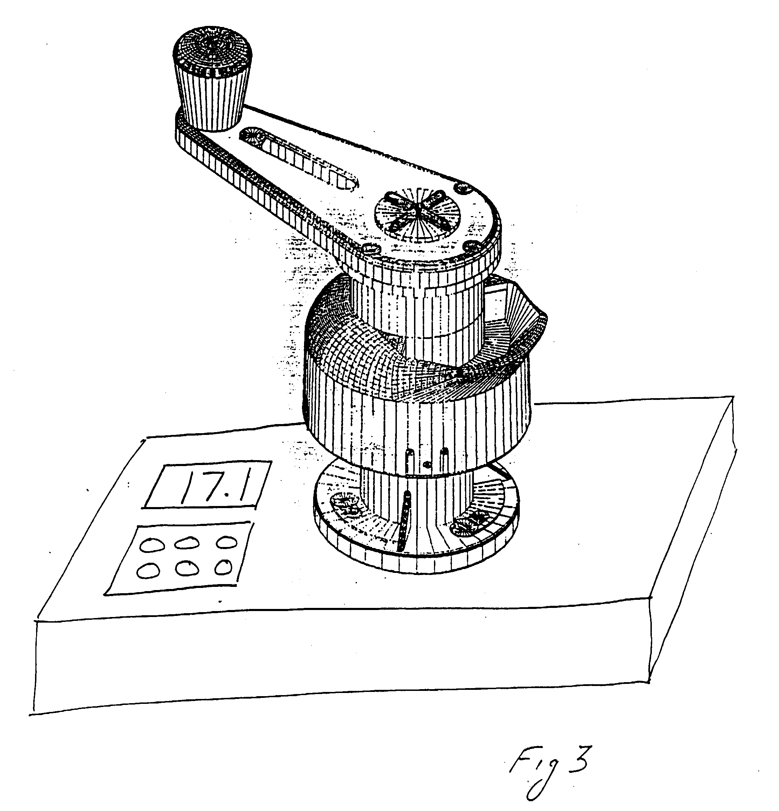

Figure 3 is a perspective view showing the grinder/compressor mounted on an electrical measuring instrument, which may be arranged to measure the electrical resistance or capacitance between the electrodes referred to and to derive from the measurement thus made a moisture content figure which may be displayed on a display provided on the instrument, for example, as illustrated, a digital LED or LCD display.

The grinding and sample-compressing apparatus may be permanently secured to the instrument (I) or may be readily detachable therefrom and re-attachable thereto, for example by means of a bayonet or screw fitting, for example for ease of cleaning. By way of example, the plate (F) with the electrodes may be permanently secured to the instrument (I) but removably inserted within the lower end of chamber (M), so that after a measurement has been made the body (A), including the peripheral wall of chamber (M) and an integral external flange at the lower end of that wall can be separated from the instrument (I) and plate (F), allowing the chamber (M) to be emptied and cleaned before the grinding and compressing apparatus is re-attached to the instrument (I).

It will be understood that after a grain sample has been ground and tested as described above, the handle (H) is rotated anti-clockwise to raise the shaft (T) and piston (D) until the shaft (T) and piston (D) are in the position shown in Figure 1, after which continued turning of the handle anti-clockwise will simply cause the clutch to slip. Thereafter the slide (G) is slid outwards along the handle, to the position shown in Figure 1, thereby decoupling the piston from the handle.

An advantage of the system described with reference to the drawings is that the grain is contained within the confines of the grinder/compressor from its raw state to its ground and compressed state and during resistance or capacitance measurement hence diminishing the chances of moisture and temperature change during grinding/transferring/compressing of the sample.

## Claims

1. Apparatus for use in measuring the moisture content of grain or other particulate material, comprising a body (A), a grinding mechanism (C) in the body for grinding grain or other particulate material, a chamber (M) provided by the body and disposed to receive, directly from the grinding mechanism, ground grain or other material ground by the grinding mechanism, a piston or ram (D) movable into said chamber (M) to compress the ground material therein, and electrical contacts so disposed in said chamber or on said ram as to be in contact with the mass of compressed ground material, resulting from such movement into the chamber, at predetermined locations on said mass, whereby the moisture content of the material can be determined on the basis of the electrical resistance, between said electrodes, provided by said mass of compressed ground material, said grinding mechanism and said ram or piston being operable selectively by a common input member (H) characterised in that said grinding mechanism (C) comprises a fixed grinding disc, stationary in the body, and a rotatable grinding disc, said common input member comprising a hub and a manually operable handle (H) secured to the hub, said hub providing the driving connection between the handle and the rotatable grinding disc, said piston or ram (D) being carried by a shaft (T) extending along the rotary axis of the handle within a hollow barrel mounted coaxially within the hub, the barrel being coupled with a cap arranged coaxially within said hub, said hollow barrel forming an output member of said clutch, and wherein (a) the shaft (T) is screw-threadedly engaged in said hollow barrel and is axially slidable in, but it non-rotatable with respect to, a central guide member fixed with respect to said body, or (b) the shaft is guided for axial movement in said barrel but is non-rotatable therein and the shaft is screw-threadedly engaged in said central guide member;
whereby rotation of said barrel within said body causes axial movement of said shaft (T) and thus of said piston (D) relative to said chamber (M), and wherein said barrel can be selectively rotationally coupled with said handle and hub or decoupled therefrom, by means of a slide (G) slidable along said handle (H) inwardly towards the rotary axis of the handle, or outwardly away from said rotary axis, respectively to extend into a diametral slot provided across the top of said cap and to be removed from said slot, and thus respectively to couple the handle with said cap and decouple the handle from said cap.

2. Apparatus according to claim 1 wherein said cap is coupled with said barrel by way of a slipping clutch within said hub, arranged to allow the cap to slip relative to the barrel when the torque exerted by the cap on the barrel exceeds a predetermined value.

3. Apparatus according to claim 2 wherein said slipping clutch comprises a first friction ring or serrated ring which is non-rotatable with respect to the cap, a second friction ring or serrated ring carried by or provided by the upper end of the barrel and a compression spring surrounding the barrel and acting to urge said first friction ring into engagement with the second friction ring.

## Patentansprüche

1. Meßgerät zur Bestimmung der Feuchtigkeit von Körnern oder anderen Teilchen, mit einem Körper (A), einem Mahlmechanismus (C) in dem Körper zum Mahlen von Körnern oder anderen Teilchen, einer Kammer (M), die von dem Körper bereitgestellt ist und zur direkten Aufhahme der gemahlenen Körner oder anderer von dem Mahlmechanismus gemahlener Teilchen von dem Mahlmechanismus angeordnet ist, einem Kolben oder Stößel (D), der zum Zusammendrücken des gemahlenen Materials in der Kammer (M) beweglich ist, und elektrischen Kontakten, die derart in der Kammer oder an dem Stößel angeordnet sind, daß sie mit der Masse des zusammengedrückten gemahlenen Materials, die aus einer derartigen Bewegung in die Kammer resultiert, an vorab bestimmten Orten auf der Masse in Kontakt stehen, wodurch die Feuchtigkeit des Materials auf der Grundlage des von der Masse aus dem zusammengedrückten gemahlenen Material bereitgestellten elektrischen Widerstands zwischen den Elektroden bestimmbar ist, wobei der Mahlmechanismus und der Stößel oder Kolben wahlweise von einem gemeinsamen Antriebsglied (H) betreibbar sind, dadurch gekennzeichnet, daß der Mahlmechanismus (C) eine feststehende Mahlscheibe, die in dem Körper stationär ist, und eine drehbare Mahlscheibe umfaßt, wobei das gemeinsame Antriebsglied eine Lagerbüchse und einen an der Lagerbüchse gesicherten, handbetätigbaren Griff (H) umfaßt, wobei die Lagerbüchse die Antriebsverbindung zwischen dem Griff und der drehbaren Mahlscheibe liefert, der Kolben oder Stößel (D) von einer sich entlang der Drehachse des Griffes in einem koaxial in der Lagerbüchse angebrachten Hohlzylinder erstreckenden Welle (T) getragen wird, der Zylinder mit einer koaxial in der Lagerbüchse angeordneten Kappe gekoppelt ist, der Hohlzylinder ein Abtriebsglied besagter Kupplung bildet, und worin (a) die Welle (T) in den Hohlzylinder geschraubt und in einem in Bezug auf den Körper feststehenden zentralen Führungsglied axial gleitfähig, aber in Bezug darauf nicht drehbar ist, oder (b) die Welle für eine Axialbewegung in dem Zylinder geführt ist, aber darin nicht drehbar ist und die Welle in das zentrale Führungsglied geschraubt ist;
wodurch eine Drehung des Zylinders in dem Körper eine Axialbewegung der Welle (T) und somit besagten Kolbens (D) relativ zu der Kammer (M) verursacht, und worin der Zylinder wahlweise mit dem Griff und der Lagerbüchse mittels einer Schiene (G) drehkoppelbar oder davon entkoppelbar ist, die entlang des Griffes (H) nach innen zu der Drehachse des Griffes oder nach außen von der Drehachse weg gleitfähig ist, um sich jeweils in einen quer über die Oberseite der Kappe vorgesehenen diametralen Schlitz zu erstrecken und um aus dem Schlitz entfernt zu werden, und somit jeweils den Griff mit der Kappe zu koppeln und den Griff von der Kappe zu entkoppeln.

2. Meßgerät nach Anspruch 1, worin die Kappe mittels einer Rutschkupplung in der Lagerbüchse, die zum Rutschenlassen der Kappe relativ zu dem Zylinder bei Überschreiten eines vorab bestimmten Wertes durch das von der Kappe auf den Zylinder ausgeübte Drehmoment angeordnet ist, mit dem Zylinder gekoppelt ist.

3. Meßgerät nach Anspruch 2, worin die Rutschkupplung einen ersten Reibring oder gekerbten Ring, der in Bezug auf die Kappe nicht drehbar ist, einen zweiten Reibring oder gekerbten Ring, der von dem oberen Ende des Zylinders getragen oder davon bereitgestellt wird, und eine Druckfeder umfaßt, die den Zylinder umgibt und den ersten Reibring in Eingriff mit dem zweiten Reibring zwingt.

## Revendications

1. Appareil à utiliser pour mesurer la teneur en humidité de graines ou d'un autre matériau particulaire, comprenant un corps (A), un mécanisme de broyage (C) disposé dans le corps pour broyer le grain ou autre matériau particulaire, une chambre (M) aménagée dans le corps et disposée de façon à recevoir, directement du mécanisme de broyage, le grain ou autre matériau broyé qui a été broyé par le mécanisme de broyage, un piston ou marteau (D) mobile dans ladite chambre (M) de façon à y comprimer ledit matériau broyé, et des contacts électriques disposés dans ladite chambre ou sur ledit marteau de façon à être en contact avec la masse de matériau broyé comprimé, du fait de ce déplacement dans la chambre, en des endroits prédéterminés de ladite masse, ce en conséquence de quoi la teneur en humidité du matériau peut être déterminée en fonction de la résistance électrique créée entre lesdites électrodes par ladite masse de matériau broyé comprimé, ledit mécanisme de broyage et ledit marteau ou piston pouvant être actionnés de manière sélective par un organe d'entrée commun (H), l'appareil étant caractérisé en ce que ledit mécanisme de broyage (C) comprend un disque de broyage fixe, stationnaire dans le corps, et un disque de broyage mobile, ledit organe d'entrée commun comprenant un moyeu et une poignée actionnable à la main (H) fixée au moyeu, ledit moyeu réalisant le raccord d'entraînement entre la poignée et le disque de broyage rotatif, ledit piston ou marteau (D) étant porté par un arbre (T) s'étendant le long de l'axe de rotation de la poignée, à l'intérieur d'un cylindre creux monté coaxialement au moyeu, le cylindre étant accouplé à un couvercle disposé coaxialement à l'intérieur dudit moyeu, ledit cylindre creux formant un organe extérieur dudit accouplement, et (a) l'arbre (T) étant vissé dans ledit cylindre creux et pouvant glisser axialement, mais sans tourner par rapport à lui, dans un organe de guidage central fixe par rapport audit corps, ou bien (b) l'arbre étant guidé de façon à se déplacer dans la direction axiale dans ledit cylindre, mais ne pouvant pas tourner dans ce dernier, et l'arbre étant vissé dans ledit organe de guidage central ;
ce en conséquence de quoi la rotation dudit cylindre dans ledit corps entraîne un déplacement axial dudit arbre (T) et donc dudit piston (D) par rapport à ladite chambre (M), ledit cylindre pouvant être sélectivement accouplé en rotation à ladite poignée et au moyeu ou bien désaccouplé de ces derniers, au moyen d'un coulisseau (G) pouvant glisser le long de ladite poignée (H) vers l'intérieur en direction de l'axe de rotation de la poignée, ou bien vers l'extérieur en s'éloignant dudit axe de rotation, pour respectivement s'étendre dans une fente diamétrale aménagée au travers de la partie supérieure dudit couvercle et être enlevé de ladite fente, et donc pour respectivement accoupler la poignée avec ledit couvercle et désaccoupler la poignée d'avec ledit couvercle.

2. Appareil selon la revendication 1, dans lequel ledit couvercle est accouplé audit cylindre au moyen d'un accouplement à glissement à l'intérieur dudit moyeu, aménagé de façon à permettre au couvercle de coulisser par rapport au cylindre lorsque le couple exercé par le couvercle sur le cylindre excède une valeur prédéterminée.

3. Appareil selon la revendication 2, dans lequel ledit accouplement à glissement comprend une première bague de friction ou bague cannelée portée ou bien réalisée par l'extrémité supérieure du cylindre, et un ressort de compression entourant le cylindre et agissant de façon à pousser ladite première bague de friction en prise avec la deuxième bague de friction.
